Europäisches Patentamt

European Patent Office

Office européen des brevets

(11) Publication number: **0 363 338**
**A2**

## EUROPEAN PATENT APPLICATION

(21) Application number: 89850323.0

(22) Date of filing: 03.10.89

(51) Int. Cl.⁵: **A61M 5/315**

(30) Priority: 03.10.88 NO 884377

(43) Date of publication of application:
**11.04.90 Bulletin 90/15**

(84) Designated Contracting States:
**AT BE CH DE ES FR GB GR IT LI LU NL SE**

(71) Applicant: HEIMREID, Ken
Jupiter Ring 41D
N-3942 Skjelsvik(NO)

(72) Inventor: HEIMREID, Ken
Jupiter Ring 41D
N-3942 Skjelsvik(NO)

(74) Representative: Stolt, Lars C. et al
L. A. Groth & Co AB Västmannagatan 43
S-113 25 Stockholm(SE)

(54) Dosing/mixing syringe.

(57) Dosing/mixing syringe comprising a barrel with a piston and a plunger where the piston and the plunger, respectively, both consist of two parts, the piston member being fixed to the plunger member which in turn is housed in and axially and rotatably movable in the plunger member which via a locking means is fixed to the piston member which in turn houses an axially and rotabably movable element consisting of a plunger with a piston head.

FIG.1

EP 0 363 338 A2

# DOSING/MIXING SYRINGE

The present invention relates to a dosing/mixing syringe for use in the health sector and preferably for use in connection with preparations, medications and bodily fluids where current demands call for maximum efficacy and precision, together with optimum safety and security.

There are a number of known devices on the market today for mixing, sampling and dosing.

Most of these are extremely complicated, and consist of many components without necessarily providing thereby any improvements with respect to hygiene and safety.

This is a problem that has occupied the person skilled in the art for many years, and as a simple summary we shall refer to devices such as, for example, the one according to DE-PS 31730 from 1884, SE-PS 142967 from 1950, and more recently, for example, US-PS 2 761 447 and 3 016 896.

From more recent material in this area, reference is made to the generally accessible Norwegian application no. 861922.

The general problem situation in this area is that the operator must be secured against unintentional or involuntary contact with the medium that is being treated. An obvious deficiency with the common commercial syringes found on the market today is that the plunger connecting the piston with the handle can come into contact with the operator's hands and then with the interior wall of the syringe, a clear path of infection that is difficult to do anything about.

In addition to providing maximum safety for both the patient and the operator, providing maximum security and precision in connection with the manipulations that are being carried out, and with an eye toward ensuring maximum safety in the later disposition of the used material, the present invention is aimed toward a dosing/ mixing syringe that comprises a barrel with a piston and the plunger associated therewith.

The syringe is characterized by the features disclosed in the main claim, the invention's syringe being characterized in that the piston and the plunger, respectively, both consist of two parts, one piston member being fixed to a plunger member which in turn is housed in and axially and rotatably movable within the second plunger member which via a locking means is fixed to the second piston member, which in turn houses an axially and rotatably movable element consisting of a plunger with a piston head.

The invention shall be illustrated further with reference to the enclosed drawings, where:

Fig. 1 shows the essential features of the invention, namely the two-part piston (1, 2) where the member (1) is connected to the plunger member (8) and the piston member (2) is connected to the the plunger member (4);

Fig. 2 shows a complete syringe before ventilation and final preparation prior to use;

Fig. 2a shows a longitudinal ridge (5) along the plunger (4) prior to ventilation and final preparation for use;

Fig. 3 shows the situation after readying the syringe for filling of the requisite cannula with liquid;

Fig. 4 shows the situation after the desired volume of liquid has been drawn into the piston barrel.

Fig. 5 shows the piston in liberated state from the situation indicated in fig. 2a with the liquid contained in the interior plunger member; and

Fig. 6 shows the situation after a completed dosage injection.

According to the invention one achieves the possibility of being able to administer exact dosages of medications which, for example, must be given intraveneously at predetermined times, for a period of up to 48 hours, and where the dosage volume can be varied from 0.01 to, for example, 10 ml.

Furthermore, the invention enables one to administer such exact dosages without having to add fluid to the syringe from a liquid source, ampule or the like, each time it is to be used. According to the present application, one would be able to administer a series of precisely measured dosages.

In accordance with the present application it would be possible for the syringe to be used for several purposes, namely for medication, for dosing, or for sampling.

According to the application as described below, one also obtains a maximum increase in safety since the sampled liquid, whether this be from a patient or from a fluid supply, is sucked into a separate and closed off chamber in the syringe without any possibility for subsequent maximum intentional contact with a subject.

The current problem in connection with syringes of this type resides in the fact that prior to further use of the syringe, air and any existing liquid is pressed out of the syringe and the cannula into free air so that there exists a complete liquid column from the piston and out into the tip of the cannula. This represents a safety risk that one does not have with the article according to the present application.

Thus, the essential feature of the application is that the final readying of the syringe for sampling or injection takes place by sucking air and a first minimum amount of liquid into the syringe where it remains during the rest of the operation. This is done by liquid being sucked in with the aid of the piston (2) through the channel (17) and into a cubicle between the piston members (1) and (2) and remaining there during the entire procedure.

The general use of the apparatus according to the invention is as follows:

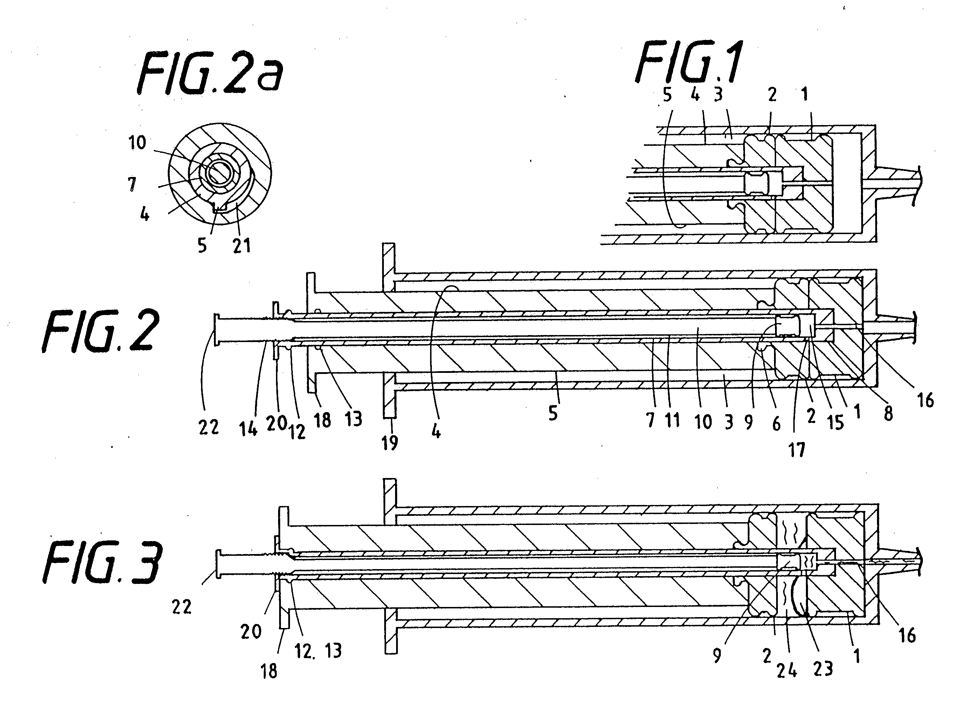

As indicated in fig. 1, there is shown a two-part piston (1, 2), somewhat retracted into the piston barrel (3), the piston prior to storage before use being to some extent attached to the walls of the exterior piston barrel (3). Immediately before use, the piston is loosened by being pressed towards and into the end wall of the piston barrel (3), as indicated in fig. 2.

Thus, in figure 2 one sees the two-part piston (1, 2) prior to the intake or expulsion of the air that is found up to the liquid which is to be introduced into the piston barrel (via a cannula).

As mentioned before, the forward member (1) of the piston is fixed to a plunger member (8).

The plunger (8) is of such a length that with the aid of a bead and groove means (12, 13) and the operation of the flanges (20) it may be locked securely into the plunger member (4).

This shall be indicated in more detail below.

When air is to be sucked out of the injection system described above, the plunger member (4) and the plunger member (8) are operated by being moved toward each other so that the bead (12) and the groove (13) are brought into engagement, which brings these two plunger members into a locked position after air from the cannula together with a small amount of liquid are brought into the intermediate chamber now formed between the piston members (1) and (2).

This is one of the essential features of the invention, inasmuch as any form of emisson from the syringe is eliminated, and the suction of liquid of any type and air from the cannula will be held in the intermediate chamber between the piston members (1) and (2) in locked position by means of the locking means (12,13).

The piston head (9) is screwed with the aid of the plunger (10) into threads (14), this unit thereby covering the channel (17) so that the air (23) and liquid (24) found between the piston members (1) and (2) is not sucked in or out, respectively, in the piston barrel (3) when the liquid (4) is sucked into the piston barrel (3) by means of the piston member (1).

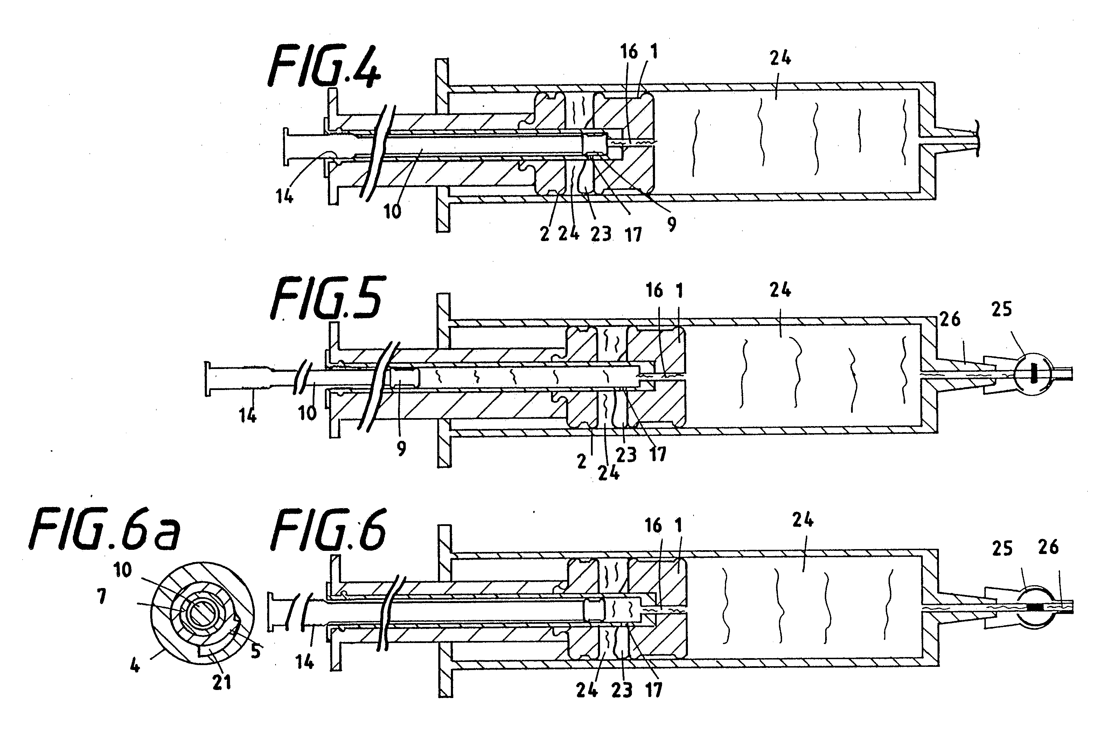

After this has been done, liquid can be sucked into the piston as indicated in fig. 4 under the reference number (24).

From this time, the syringe can be used as an elementary syringe in that the innermost piston (10) with head (9) can be locked in the upper apparatus; in this case the liquid (9) is pressed out in the usual manner by means of operation of the piston unit comprising the members (1, 2) and (8, 4), respectively.

In the meantime, by releasing the locking (14) of the piston (10) with the head (9), one obtains the possibility of determining precise dosages by allowing the unit (10, 9) to act as a floating piston in the plunger member (8). On pressing the plunger member (4) against the sucked-in fluid (24), the piston head (9) with its associated plunger (10) is pushed back.

This is made possible with the aid of a valve means (25) which, in accordance with fig. 5, is in the closed position when liquid is being pressed into the plunger member (8) where the reading of an amount of pressed liquid is done by means of the piston head and the plunger (9, 10).

On injection, the valve (25) is then opened, and the piston (10) with the head (9) is operated while the remaining unit is locked as indicated in fig. 6a by means of the ridge (5) in a groove (21). This makes it possible to obtain a totally exact dosage of the liquid that is to be emitted.

With subsequent dosing of injection volumes, one can achieve the same result with the aid of the valve means (25) and the freely floating piston element (9, 10).

The subject of the invention can also be used as a mixing-dosing device, in that the syringe with reference to fig. 3 is delivered with a liquid or dry matter already inserted between the pistons (1) and (2). In this case the piston (9) is screwed securely into the bottom of the piston barrel (11) so that the channel (17) is closed and shut off. The plunger (4) in this case is locked with the above mentioned bead or ridge (5) as indicated in fig. 6a.

To introduce liquid into the piston barrel (3), the element (5) is set in the free position as shown in fig. 2a.

In fig. 4, the liquid (2) is indicated with a correct volume relationship between dry matter or liquid between the pistons (1) and (2). The air and the previously mentioned excess liquid that is found in the piston barrel (3) is at this time pressed out of the barrel (26), out of the tap (25) and out of the cannula (not shown).

For dosing purposes, the tap (25) is then closed, the piston (9) is screwed slightly back so that the channel opening (17) is free for the transfer of some liquid from the piston barrel (3) to dry matter/liquid for dissolving or mixing.

The further injection/administration procedure will be apparent from what is stated hereinabove.

As mentioned introductorily, by means of the apparatus according to the present application one would obtain maximum safety with regard to operation and hygiene with a minimum of complications, since the syringe is easily operated and safe.

The device, with the assistance of simple aids, can give exact dosages, and the safety aspect is taken care of particularly by the feature that sampled liquid or other fluid is sucked into the syringe and not, as today, pressed out.

This means that any potential injurious agents, compounds or liquids are, to a greater extent that has been achieved thus far, removed from possible contact that can be injurious to health.

## Claims

1. A dosing/mixing syringe comprising a barrel (3) with a piston (1, 2) and a plunger (8, 4), **characterized in** that the piston and the plunger, respectively, both consist of two parts, one piston member (1) being fixed to a plunger member (8) which in turn is housed in and axially and rotatably movable within the second plunger member (4) which via a locking means (6) is fixed to the second piston member (2) which in turn houses an axially and rotatably movable element (22) consisting of a plunger (10) with a piston head (9).

2. A syringe according to claim 1, **characterized in** that it has a tapered end (26) adapted for housing of a valve means (25).

3. A syringe according to claim 1, **characterized in** that it is equipped with a locking means with rotatable locking of the plunger member 4 with the aid of a ridge 5 in a conical tapered groove 21.

4. A syringe according to any one of the preceding claims, **characterized** by means (12, 13) for locking of the plunger member (8) to the plunger member (4).

5. A syringe according to any one of the preceding claims, **characterized in** that the floating piston (10) with head (9) is equipped with locking means (14) for attachment to the plunger member (8).

FIG.2a

FIG.1

FIG.2

FIG.3

EP 0 363 338 A2

FIG.4

FIG.5

FIG.6

FIG.6a